# EUROPÄISCHE PATENTANMELDUNG

(11) **EP 2 130 561 A1**
(43) Veröffentlichungstag der Anmeldung: **09.12.2009**
(21) Anmeldenummer: 09007454.3
(22) Anmeldetag: 05.06.2009
(51) Int. Cl.: A61M 5/315, A61M 5/00, A61M 5/31

(54) **Medizinische Spritze mit Kolbensperrglied**

(30) Priorität: 06.06.2008 DE 102008028211
(71) Anmelder: Aesculap AG, 78532 Tuttlingen/Donau (DE)
(72) Erfinder: Odermatt, Erich, 8200 Schaffhausen (CH); Löffler, Burkhard, 79868 Feldberg (Falkau) (DE); König, Silke, 78628 Rottweil (DE)
(74) Vertreter: Renger, Florian

(57) **Zusammenfassung**

Die Erfindung betrifft eine medizinische Spritze (1) mit einem Spritzenzylinder (2) aus Kunststoff mit einer reversibel verschlossenen Austragsöffnung (3) an seiner Vorderseite und einem im Zylinder angeordneten Kolben (8), der mit einem aus dem Spritzenzylinder axial herausragenden als Betätigungsglied dienenden Stößel (9) verbunden ist, wobei der Spritzenzylinder (2) mindestens teilweise mit einem sich bei Erwärmung ausdehnenden wässrigen Medium (11) gefüllt ist, wobei der Spritze (1) ein als gesondertes Bauteil ausgebildetes Sperrglied (41) zugeordnet ist, das ein Herausdrücken des Kolbens (8) bei Anwendung von Temperaturen über dem Siedepunkt des wässrigen Mediums (11) verhindert.

## Beschreibung

Die Erfindung betrifft eine medizinische Spritze mit einem Spritzenzylinder aus Kunststoff mit einer reversibel verschlossenen Austragsöffnung an seiner Vorderseite und einem im Zylinder angeordneten Kolben, der mit einem aus dem Spritzenzylinder axial herausragenden als Betätigungsglied dienenden Stößel verbunden ist, und wobei der Spritzenzylinder mindestens teilweise mit einem sich bei Erwärmung ausdehnenden wässrigen Medium gefüllt ist.

Bei chirurgischen Eingriffen, insbesondere bei der minimalinvasiven Chirurgie, werden häufig kleine Mengen an sterilen Flüssigkeiten auf wässriger Basis eingesetzt. Die Flüssigkeiten werden entweder direkt an der Eingriffsstelle ausgegeben oder, insbesondere bei der minimalinvasiven Chirurgie, durch ein Endoskop und Laparoskop beispielsweise in den Bauchraum ausgegeben. Bei den wässrigen Medien bzw. Flüssigkeiten handelt es sich insbesondere um flüssige Mittel zur Adhäsionsprophylaxe oder auch um flüssige Klebstoffe auf wässriger Basis. In der Regel werden solche wässrigen Medien in größeren Gebinden vorrätig gehalten, die mit einem mittels einer Spritzennadel durchstechbaren Stopfen versehen sind. Das wiederholte Entnehmen kleinerer Mengen aus solchen Gebinden ist einerseits arbeitsaufwendig. Auf der anderen Seite ist die sterile Entnahme nicht immer gewährleistet.

Andererseits kann es vorgesehen sein, derartige wässrige Medien direkt in einer medizinischen Spritze zu bevorraten, aus der sie dann bei Bedarf problemlos ausgegeben werden können. Spritze und Inhalt müssen jedoch in steriler Form vorliegen. Dies bedeutet, daß die Spritze nach ihrer Befüllung und dem Verschließen der Befüllöffnung samt Inhalt sterilisiert wird, was normalerweise bei 121 °C in Dampf vorgenommen wird. Bei diesen Temperaturen ist der Siedepunkt des wässrigen Mediums in aller Regel überschritten. Hinzukommt noch die hydraulische Ausdehnung des wässrigen Mediums. Deshalb besteht die Gefahr, daß der Kolben während der Erwärmung aus dem Spritzenzylinder herausgedrückt wird und der Inhalt verlorengeht bzw. andere Spritzen kontaminiert. Die Spritzen könnten zwar im Sterilisator eingespannt werden, um ein solches Herausdrücken des Kolbens zu vermeiden. Das Entnehmen der Spritzen aus dem Sterilisator und die Verpackung der Spritzen sind jedoch mit der Gefahr verbunden, daß die Sterilität verlorengeht.

Mitte des letzten Jahrhunderts wurden noch wiederverwendbare Spritzen verwendet, bei denen der Spritzenzylinder aus Glas bestand und der Spritzenkolben aus Metall. Derartige Spritzen waren zur Sterilisation zerlegbar und hatten am hinteren Ende des Spritzenzylinders einen Bajonettverschluß, nach dessen Abnehmen der metallische Kolben aus dem Zylinder herausführbar war, damit die einzelnen Teile der wiederverwendbaren Spritze getrennt voneinander sterilisiert werden konnten.

Heutzutage handelt es sich jedoch bei den in Rede stehenden Spritzen um Einmalspritzen, die handelsüblich sind. Bevorzugt sind qualitativ hochwertige Spritzen mit einem Kolben aus Gummi und einer Zylinderwandung aus einem Cycloolefin-Copolymer aus Norbornen und Ethylen, das unter der Handelsbezeichnung TOPAS (Thermoplastic Olefin Polymer of Amorphous Structure) bekannt ist. Einfache Einmalspritzen, die vollständig aus Kunststoff bestehen und einen tellerförmigen Kolben aus Kunststoff besitzen, der einstückig mit dem Stößel durch Spritzguß gefertigt ist, besitzen zwar häufig am hinteren Ende des Spritzenzylinders eine Einschnürung, die den lichten Durchmesser des Spritzenzylinders verjüngt. Eine solche Einschnürung soll ein versehentliches Herausziehen des Kolbens aus dem Spritzenzylinder vermeiden, ist jedoch als Sperrglied für die hier vorgesehenen Zwecke nicht geeignet, weil die Einschnürung vom Kolben bei Anwendung größerer Kräfte durchfahren werden kann.

Dementsprechend stellt sich die Erfindung die Aufgabe, eine alternative Möglichkeit bereitzustellen, damit die gefüllte Spritze sicher sterilisiert werden kann, vorzugsweise in ihrer Verpackung. Dabei soll ein Herausdrücken des Kolbens der medizinischen Spritze bei Anwendung von Temperaturen über den Siedepunkt des wässrigen Mediums zuverlässig verhindert werden.

Diese Aufgabe wird gelöst durch den Gegenstand des Anspruchs 1. Bevorzugte Ausführungsformen dieses Gegenstandes sind in den abhängigen Ansprüchen 2 bis 14 dargestellt. Anspruch 15 betrifft ein Sperrglied für eine medizinische Spritze, das zur Lösung der erfindungsgemäßen Aufgabe in besonderer Weise geeignet ist.
Der Wortlaut sämtlicher Ansprüche wird hiermit durch Bezugnahme zum Inhalt dieser Beschreibung gemacht.

Erfindungsgemäß ist der eingangs beschriebenen medizinischen Spritze ein Sperrglied zugeordnet, das ein Herausdrücken des Kolbens der Spritze bei Anwendung von Temperaturen über den Siedepunkt des wässrigen Mediums verhindert. Das Sperrglied ist dabei als gesondertes Bauteil ausgebildet.

Vorzugsweise ist das Sperrglied mit der Spritze lösbar verbunden, so dass es bei Gebrauch der Spritze in einfacher Weise wieder von der Spritze getrennt werden kann.
Vorzugsweise ist dabei das Sperrglied mit der Spritze verriegelbar, so dass das Lösen durch ein Entriegeln erfolgt.

In Weiterbildung ist die mit dem wässrigen Medium gefüllte Spritze zusammen mit dem Sperrglied in mindestens einer sterilen Verpackung untergebracht. Auf diese Weise wird die Spritze für den Gebrauch sterilisiert bereitgestellt. Insbesondere liegen die Spritze und das Sperrglied erfindungsgemäß bereits während der Sterilisation in mindestens einer Verpackung vor, wobei dann Spritze, Sperrglied und Verpackung gemeinsam sterilisiert werden.

Für die Funktionsweise des Sperrglieds bzw. für das Zusammenwirken von Sperrglied und Spritze gibt es verschiedene Möglichkeiten. Bei einer bevorzugten Ausführungsform der Erfindung steht das Sperrglied in Wirkverbindung mit dem Zylinder und dem Stößel, insbesondere in Wirkverbindung mit Anschlagflächen von Zylinder und Stößel. Weiterhin ist es erfindungsgemäß bevorzugt, dass das Sperrglied mindestens zum Teil, vorzugsweise ausschließlich, formschlüssig sperrt. Dies bedeutet, dass das verriegelte Sperrglied nur durch Veränderung bzw. Bruch außer Kraft gesetzt werden kann. Dadurch hat das Sperrglied gleichzeitig auch die Funktion einer Originalitätssicherung.

Es ist bei einer bevorzugten Ausführungsform der Erfindung möglich, dass das Sperrglied eine in Richtung zur Austragsöffnung weisende Fläche des Spritzenzylinders und/oder eine in entgegengesetzter Richtung weisende Fläche des Stößels hintergreift. So kann das Sperrglied an einem an der Rückseite des Spritzenzylinders vorgesehenen Flansch angreifen. Es kann aber auch an der vorderen Verschlußseite des Spritzenzylinders angreifen.

Andererseits ist es möglich, dass das Sperrglied an der Rückseite des Stößels, insbesondere an einer hinteren Druckplatte des Stößels, angreift. Es ist aber auch möglich, dass das Sperrglied an der Rückseite des Kolbens angreift, soweit der Durchmesser des Kolbens größer ist als die entsprechenden Ausmaße des Stößels.

Ein derartiger Wirkmechanismus zwischen Sperrglied und Spritze ermöglicht es, handelsübliche Einmalspritzen zu verwenden, ohne dass an diesen irgendwelche baulichen Veränderungen vorzunehmen sind.

Das Sperrglied kann auch von einem Kunststoffteil, insbesondere einem Spritzgußteil, gebildet werden, das Einrichtungen zum Hintergreifen von Anschlagflächen des Spritzenzylinders und des Stößels aufweist. Um das Sperrglied in seiner baulichen Größe zu begrenzen, ist es vorzugsweise so ausgebildet, dass es eine zur Austragsöffnung gerichtete Fläche eines an der Rückseite des Spritzenzylinders vorgesehenen Flansches einerseits hintergreift und eine hintere Fläche des Stößels, insbesondere die Rückseite einer Druckplatte des Stößels. Es ist aber auch möglich, dass die Länge des Sperrglieds im wesentlichen der Länge der gefüllten Spritze entspricht und an der Vorderseite der Spritze einerseits und der Rückseite des Stößels andererseits angreift.

Das Sperrglied hat gemäß einer bevorzugten Ausführungsform zusätzlich zu seiner Sperrfunktion vorzugsweise noch eine Klemmfunktion, durch die es im aufgebrachten Zustand an der Spritze verbleibt, d.h. gegen ein versehentliches Entfernen gesichert ist. Weiterhin kann das Sperrglied mit Vorteil so ausgebildet sein, dass es in Bezug auf die sperrende Funktion ein gewisses Spiel besitzt, das die hydraulische Volumenausdehnung des flüssigen Mediums beim Erwärmen zu kompensieren vermag.

Bei bevorzugten Ausführungsformen der Erfindung ist das Sperrglied so ausgebildet, dass es im Gebrauchszustand, d.h. wenn es seine Sperrfunktion erfüllt, ein oberes Teil/Oberteil und ein unteres Teil/Unterteil aufweist. Dieses Oberteil und dieses Unterteil sind dazu vorgesehen, die Spritze, insbesondere deren hinteren Teil mindestens teilweise, vorzugsweise vollständig zu umschließen. Dieses Umschließen erfolgt derart, dass durch entsprechende Elemente am Oberteil und/oder am Unterteil eine Festlegung bzw. ein Sperren des Kolbens gegen eine Verschiebung erfolgt. Insbesondere kann es sich hier um eine Art Arretierung oder Verriegelung des Kolbens handeln, so wie dies in den vorherigen Abschnitten bereits erläutert wurde.

Vorzugsweise können das Oberteil und/oder das Unterteil bei solchen Ausführungen nach Art einer Schale ausgebildet sein, wobei solche Schalenelemente den Spritzenkörper mindestens teilweise umschließen. Vorzugsweise ist das Sperrglied dann so ausgestaltet, dass Oberteil und Unterteil die Spritze bzw. den entsprechenden Teil der Spritze wie zwei Halbschalen umschließen.

In diesem Zusammenhang ist es bei der Erfindung vorstellbar, dass Oberteil und Unterteil zunächst voneinander getrennt sind, und dann zu einem Sperrglied miteinander verbunden werden. Dies kann beispielsweise mit Hilfe von Verbindungselementen, insbesondere Rastelementen geschehen, die an Oberteil bzw. Unterteil vorgesehen sind.

Es ist allerdings bevorzugt, wenn das Sperrglied einstückig ausgebildet ist. Dies kann die Verbindung des Sperrglieds mit der Spritze vereinfachen. Ein solches einstückiges Sperrglied lässt sich beispielsweise dadurch realisieren, dass Oberteil und Unterteil über einen oder mehrere Stege miteinander verbunden sind. Solche Versionen lassen sich dann gleich bei der Herstellung des Sperrglieds realisieren, insbesondere dann, wenn das Sperrglied, wie bei der Erfindung bevorzugt, aus Kunststoff gefertigt ist.

Die bevorzugt genannten Sperrglieder lassen sich erfindungsgemäß in einfacher Weise mit Hilfe sogenannter Blister bzw. Blisterverpackungen bereitstellen. Derartige Blister, die häufig aus Kunststoff gefertigt werden, sind dem Fachmann ohne Weiteres bekannt. Für eine einstückige Version mit Oberteil, Unterteil und Steg lassen sich erfindungsgemäß mit Vorteil sogenannte Klappblister einsetzen, d.h. (zusammen)klappbare Kunststoffteile, die aus (in der Regel transparentem) Kunststoff tiefgezogen werden.

Bei den vorstehend beschriebenen Ausführungsformen der Erfindung ist die handelsübliche Spritze ohne jegliche Veränderungen einsetzbar. Es sind aber auch Ausführungsformen möglich, bei denen zwar handelsübliche Spritzen einsetzbar sind, an diesen jedoch zum Anbringen des Sperrgliedes Veränderungen vorgenommen sind. Solche Veränderungen können an der Spritze nachträglich vorgenommen werden oder bereits bei ihrer Fertigung.

Die Austragsöffnung der Spritze an der vorderen Stirnwand des Spritzenzylinders ist mit Vorteil formschlüssig verschließbar, da der übrige Konus bei den einfachen Einmalspritzen nicht die gewünschte Sicherheit bietet. Als Verschluß kommen insbesondere ein Schraubverschluß, ein Bajonettverschluß oder der sogenannte Lurverschluß in Frage.

Weitere Merkmale und Vorteile der Erfindung ergeben sich aus der nachfolgenden Beschreibung von bevorzugten Ausführungsformen in Verbindung mit den Unteransprüchen und der Zeichnung. Hierbei können die einzelnen Merkmale jeweils für sich oder in Kombination miteinander verwirklicht sein.

In der Zeichnung zeigen:
- Figur 1:: eine medizinische Spritze nach dem Stand der Technik zum Einsatz bei der Erfindung.
- Figur 2:: eine Kombination aus einer medizinischen Spritze und einem Spritzenstabilisator (Sperrglied) gemäß der Erfindung,
- Figuren 3 und 4:: den Spritzenstabilisator nach Figur 7 in Draufsicht und Seitenansicht, und
- Figur 5:: die in Figur 2 dargestellte Kombination in steril verpackter Form.

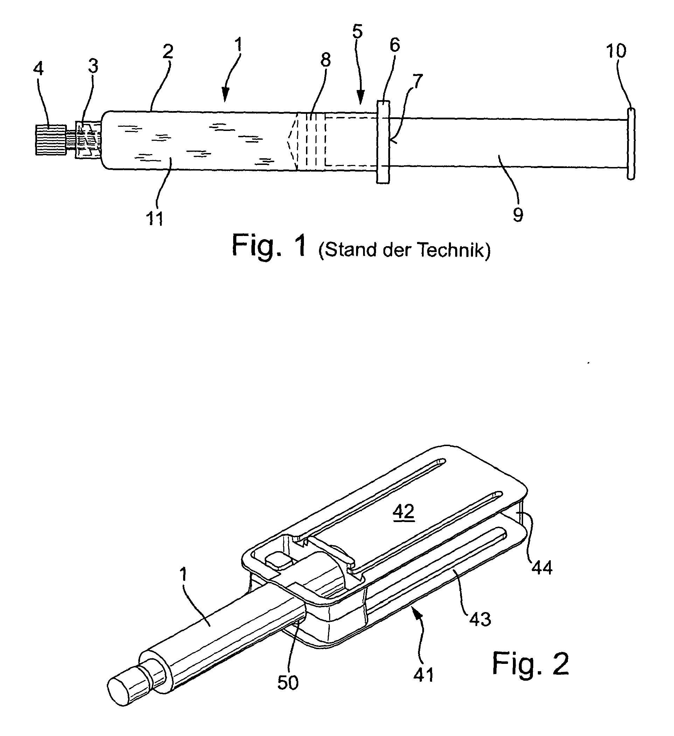

In Figur 1 ist eine handelsübliche Spritze zum erfindungsgemäßen Zusammenwirken mit einem Spritzenstabilisator (Sperrglied) dargestellt. Die Spritze 1 weist einen Spritzenzylinder 2 auf, der an seinem vorderen Ende eine Ausgabeöffnung 3 in Form eines rohrförmigen Stutzens mit einem Innengewinde besitzt. Die Öffnung 3 ist mit einem eingedrehtem Stopfen 4 verschlossen. Am hinteren Ende 5 weist der Spritzenzylinder 2 einen Flansch 6 auf, der eine Öffnung 7 umgibt, die dem Innendurchmesser des Zylinders 2 entspricht. In den Zylinder 2 ist ein verschiebbarer Kolben 8 eingeschoben, der bei dieser Ausführungsform aus Gummi besteht. Der Kolben 8 ist mit einem aus der Öffnung herausragenden Stößel 9 verbunden, der einen kreuzförmigen Querschnitt besitzt und an seinem freien Ende eine Abschlussplatte 10 aufweist. Der Spritzenzylinder 2 und der Stößel 9 bestehen aus Kunststoff. Der Zylinder 2 ist mit einer zu sterilisierenden wässrigen Flüssigkeit, insbesondere einem Hydrogel, beispielsweise eine Suspension/Lösung von Polyvinylalkohol (PVA) gefüllt. Diese wässrige Flüssigkeit soll in der Spritze 1 bei Temperaturen von 121 °C sterilisiert werden. Das Volumen der wässrigen Flüssigkeit beträgt normalerweise zwischen 2 und 20 ml. Bei der handelsüblichen Spritze handelt es sich um eine Einmalspritze.

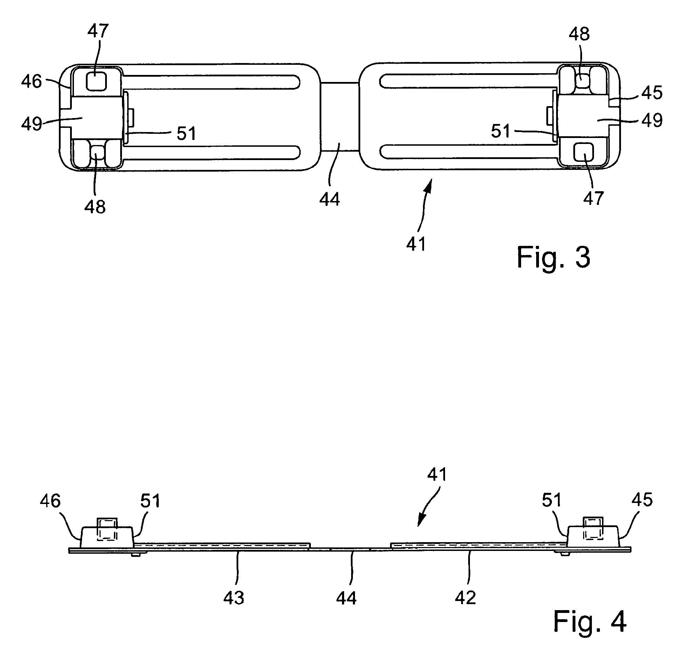

Der Spritze 1 wird erfindungsgemäß ein Sperrglied zugeordnet. Dies ist in den folgenden Figuren 2 bis 5 dargestellt.

Es ist bei allen Ausführungsformen der Erfindung bevorzugt, dass die Kombination aus Spritze und Spritzenstabilisator bzw. Sperrglied während der Sterilisation vollständig verpackt ist. Dies ermöglicht es, die Spritze steril aus dem Sterilisator zu entnehmen. Der Spritzensterilisator bzw. das Sperrglied wird erst unmittelbar vor dem Einsatz der Spritze von dieser entfernt.

Bei der Ausführungsform nach den Figuren 2 bis 4 ist eine Spritze vorgesehen, die der Spritze 1 nach Figur 1 entspricht. Dabei dient bei dieser Ausführungsform ein Spritzenstabilisator 41 als Sperrglied, welcher nur einen Teil der Spritze 1, nämlich den hinteren Teil umfasst. Der Begriff "Spritzenstabilisator" soll dabei zum Ausdruck bringen, dass dieser die Lage des Kolbens in der Spritze, wie gewünscht, festlegt, d.h. stabilisiert. Bei dem Spritzenstabilisator 41 handelt es sich um einen Klappblister, der als klappbares Tiefziehteil einstückig ausgebildet ist. Eine obere Hälfte 42 ist mit einer unteren Hälfte 43 über einen als Rückwand dienenen Steg 44 verbunden. Die Teile 42 und 43 sind gegeneinander klappbar, wie dies in Figur 2 gezeigt ist. Oberes Teil 42 und unteres Teil 43 besitzen an ihren freien Enden aus der Ebene des Klappblisters hochgezogene Erhöhungen 45 bzw. 46. Diese Erhöhungen haben an ihren Seiten zueinander passende Vorsprünge 47 bzw Vertiefungen 48, die ineinander passen und eine Rastverbindung zum Schließen des Klappblisters 41 bilden. Zwischen den Vorsprüngen 47 und Vertiefungen 48 befinden sich halbkreisförmige Senken 49, die zusammen eine kreisrunde Öffnung 50 ergeben, durch die der Zylinder 2 der Spritze 1 passt. Die bei geöffnetem Blister zueinander weisenden Innenseiten 51 der Erhöhungen 45 und 46 dienen in zusammengeklapptem Zustand als gemeinsamer Anschlag für die Vorderseite des Flansches 6 des Spritzenzylinders 2 (siehe Figur 1). Der hintere Anschlag wird von der als Verbindungsstück dienenden Rückwand 44 des Klappblisters gebildet.

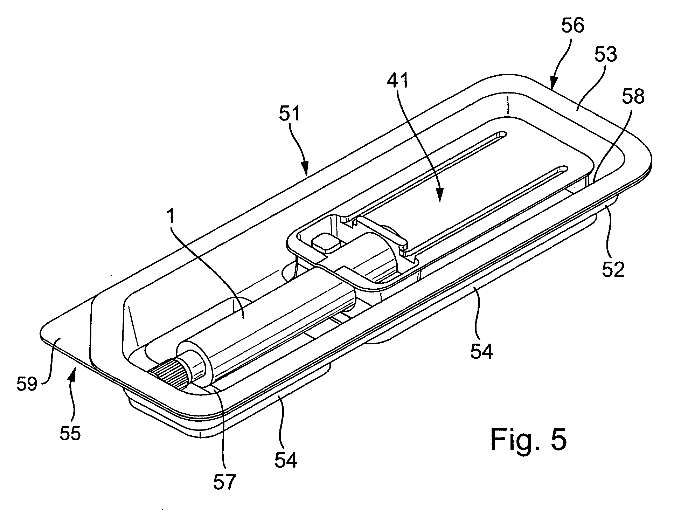

Da der Klappblister 41 bei dieser Ausführungsform nicht die Funktion einer vollständigen Verpackung erfüllt, ist hier eine gesonderte Verpackung vorgesehen. Diese kann ihrerseits als Blisterverpackung ausgebildet sein, was in Figur 5 dargestellt ist.

In Figur 5 ist eine Verpackung 51 dargestellt, die die in Figur 2 dargestellte Ausführungsform der Erfindung aus Spritze 1 und Sperrglied (Spritzenstabilisator) 41 enthält.

Die Verpackung 51 besteht im Wesentlichen aus zwei Teilen, nämlich einer die Spritze 1 und das Sperrglied 41 aufnehmenden Schale 52, die man auch als Unterteil bezeichnen kann, und einem ebenen Deckel, der in der Zeichnung nicht dargestellt ist. Dieser Deckel ist mit einem umlaufenden Rand 53 der Schale 52 zum Verschließen der Verpackung 51 verschweißbar. Der Deckel kann beispielsweise aus gasdurchlässigem, aber keimdichtem Papier oder Vlies bestehen, das z.B. unter dem Namen Tyvec^{®} im Handel erhältlich ist. Schale 52 und Deckel sind nach Art einer Blisterpackung ausgebildet. Der mit der Schale 52 verschweißte Deckel stabilisiert die Schale 52 mechanisch, so dass diese aus relativ dünnem Kunststoff gefertigt werden kann.

Gemäß Figur 5 besitzt die Schale 52 im Bereich ihres Bodens Abstufungen bzw. Vertiefungen 54 und an ihrer vorderen Schmalseite 55 und ihrer hinteren Schmalseite 56 entsprechende Abstufungen bzw. Ausnehmungen 57 bzw. 58. Diese dienen einerseits zur mechanischen Verstärkung der Schale 52, und andererseits als Auflageflächen für die Spritze 1 bzw. das Sperrglied 41. Sie können auch zusätzlich zur Festlegung von Spritze 1 und Sperrglied 41 in der Verpackung 51 dienen und damit unter Umständen zusätzlich zur Funktion des Sperrglieds 41 eine von der Erfindung nicht gewünschte Bewegung des Kolbens in der Spritze verhindern.

In diesem Zusammenhang legt die Ausnehmung 57 an der Schmalseite 55 das vordere Ende der Spritze 1 fest, und die Ausnehmung 58 am hinteren Ende 56 das hintere Ende des Sperrglieds 41.

Schließlich sei erwähnt, dass der umlaufende Rand 53 an der Schale 52 an einer Ecke der Schale 52 abgeschrägt ist. Dadurch wird eine Öffnungslasche 59 gebildet, die ein Öffnen der Verpackung 51 erleichtert.

## Patentansprüche

1. Medizinische Spritze (1) mit einem Spritzenzylinder (2) aus Kunststoff mit einer reversibel verschlossenen Austragsöffnung (3) an seiner Vorderseite und einem im Zylinder angeordneten Kolben (8), der mit einem aus dem Spritzenzylinder axial herausragenden als Betätigungsglied dienenden Stößel (9) verbunden ist, und wobei der Spritzenzylinder (2) mindestens teilweise mit einem sich bei Erwärmung ausdehnenden wässrigen Medium (11) gefüllt ist, **dadurch gekennzeichnet, dass** der Spritze (1) ein als gesondertes Bauteil ausgebildetes Sperrglied (41) zugeordnet ist, das ein Herausdrücken des Kolbens (8) bei Anwendung von Temperaturen über dem Siedepunkt des wässrigen Mediums (11) verhindert.

2. Medizinische Spritze nach Anspruch 1, **dadurch gekennzeichnet, dass** das Sperrglied (41) mit der Spritze lösbar verbunden ist.

3. Medizinische Spritze nach Anspruch 1 oder Anspruch 2, **dadurch gekennzeichnet, dass** die mit dem wässrigen Medium (11) gefüllte Spritze (1) zusammen mit dem Sperrglied (41) in mindestens einer sterilen Verpackung vorliegt.

4. Medizinische Spritze nach einem der vorhergehenden Ansprüche, **dadurch gekennzeichnet, dass** das Sperrglied (41) in Wirkverbindung mit dem Zylinder und dem Stößel, insbesondere in Wirkverbindung mit Anschlagflächen (44; 51) von Zylinder und Stößel, steht.

5. Medizinische Spritze nach einem der vorhergehenden Ansprüche, **dadurch gekennzeichnet, dass** das Sperrglied (41) mindestens zum Teil formschlüssig sperrt.

6. Medizinische Spritze nach einem der vorhergehenden Ansprüche, **dadurch gekennzeichnet, dass** das Sperrglied (41) eine in Richtung zur Austragsöffnung weisende Fläche des Spritzenzylinders und/oder eine in entgegengesetzter Richtung weisende Fläche des Stößels hintergreift.

7. Medizinische Spritze nach einem der vorhergehenden Ansprüche, **dadurch gekennzeichnet, dass** das Sperrglied (41) an einem an der Rückseite des Spritzenzylinders vorgesehenen Flansch (6) angreift.

8. Medizinische Spritze nach einem der vorhergehenden Ansprüche, **dadurch gekennzeichnet, dass** das Sperrglied (41) an der Rückseite des Stößels (9) angreift.

9. Medizinische Spritze nach einem der vorhergehenden Ansprüche, **dadurch gekennzeichnet, dass** das Sperrglied von einem Kunststoffteil (20), insbesondere einem Spritzgußteil, gebildet wird, das Einrichtungen zum Hintergreifen von Anschlagflächen des Spritzenzylinders (2) und des Stößels (9) aufweist.

10. Medizinische Spritze nach einem der vorhergehenden Ansprüche, insbesondere nach Anspruch 9, **dadurch gekennzeichnet, dass** das Sperrglied (41) ein insbesondere schalenartiges Oberteil (42) und ein insbesondere schalenartiges Unterteil (43) aufweist, und diese beiden Teile (42, 43) dazu vorgesehen sind, die Spritze (1) mindestens teilweise, insbesondere mindestens deren hinteren Teil, zu umschließen, wobei vorzugsweise das Oberteil (42) und das Unterteil (43) über einen Steg (44) zu einem einstückigen Sperrglied (41) verbunden sind.

11. Medizinische Spritze nach einem der vorhergehenden Ansprüche, **dadurch gekennzeichnet, dass** die Spritze (1) eine handelsübliche Spritze ist ohne besondere Anpassungen zum Zusammenwirken mit dem Sperrglied (41).

12. Medizinische Spritze nach einem der vorhergehenden Ansprüche, **dadurch gekennzeichnet, dass** die Austragsöffnung formschlüssig verschlossen ist, insbesondere über einen Gewinde- oder Bajonettverschluss.

13. Medizinische Spritze nach einem der vorhergehenden Ansprüche, **dadurch gekennzeichnet, dass** die vom Sperrglied ausgeübte Sperre zumindest dem Dampfdruck der erwärmten Flüssigkeit entgegenwirkt.

14. Medizinische Spritze nach einem der vorhergehenden Ansprüche, **dadurch gekennzeichnet, dass** die vom Sperrglied ausgeübte Sperre ein Spiel besitzt, das den hydraulischen Druck der Flüssigkeit bei der Erwärmung kompensiert.

15. Sperrglied (41) für eine medizinische Spritze (1), die einen Spritzenzylinder (2) aus Kunststoff mit einer reversibel verschlossenen Austragsöffnung (3) an seiner Vorderseite und einen im Zylinder angeordneten Kolben (8), der mit einem aus dem Spritzenzylinder axial herausragenden als Betätigungsglied dienenden Stößel (9) verbunden ist, aufweist, und wobei der Spritzenzylinder (2) mindestens teilweise mit einem sich bei Erwärmung ausdehnenden wässrigen Medium (11) gefüllt ist, **dadurch gekennzeichnet, dass** das Sperrglied (41) ein insbesondere schalenartiges Oberteil (42) und ein insbesondere schalenartiges Unterteil (43) aufweist, wobei diese beiden Teile (42, 43) dazu vorgesehen sind, die Spritze (1) mindestens teilweise, insbesondere mindestens deren hinteren Teil, zu umschließen, wobei vorzugsweise das Oberteil (42) und das Unterteil 43) über einen Steg (44) zu einem einstückigen Sperrglied (41) verbunden sind.
